(19) **Europäisches Patentamt**
**European Patent Office**
**Office européen des brevets**

(11) **EP 1 492 531 B3**

(12) **NOUVEAU FASCICULE DE BREVET EUROPEEN**
Après la procédure de limitation (B3-1)

(45) Date de publication et mention de la décision de limitation:
**B3-1 11.04.2012 Bulletin 2012/15**

(45) Mention de la délivrance du brevet:
**10.09.2008 Bulletin 2008/37**

(21) Numéro de dépôt: **03745831.2**

(22) Date de dépôt: **07.04.2003**

(51) Int Cl.:
*A61K 31/43* (2006.01)     *A61K 9/50* (2006.01)
*A61P 31/04* (2006.01)

(86) Numéro de dépôt international:
**PCT/FR2003/001095**

(87) Numéro de publication internationale:
**WO 2003/084517 (16.10.2003 Gazette 2003/42)**

(54) **FORMULATION PHARMACEUTIQUE ORALE SOUS FORME DE SUSPENSION AQUEUSE DE MICROCAPSULES PERMETTANT LA LIBERATION MODIFIÉE D'AMOXICILLINE**

ORALE PHARMAZEUTISCHE FORMULIERUNG IN FORM EINER WÄSSRIGEN SUSPENSION VON MIKROKAPSELN ZUR MODIFIZIERTEN FREISETZUNG VON AMOXICILLIN

ORAL PHARMACEUTICAL FORMULATION IN THE FORM OF AQUEOUS SUSPENSION OF MICROCAPSULES FOR MODIFIED RELEASE OF AMOXICILLIN

(84) Etats contractants désignés:
**AT BE BG CH CY CZ DE DK EE ES FI FR GB GR HU IE IT LI LU MC NL PT RO SE SI SK TR**

(30) Priorité: **09.04.2002 FR 0204409**
**02.09.2002 FR 0210846**

(43) Date de publication de la demande:
**05.01.2005 Bulletin 2005/01**

(73) Titulaire: **FLAMEL TECHNOLOGIES**
**69200 Vénissieux (FR)**

(72) Inventeurs:
• **CASTAN, Catherine**
**69530 Olienas (FR)**
• **GUIMBERTEAU, Florence**
**F-33450 Montussan (FR)**
• **MEYRUEIX, Rémi**
**69009 Lyon (FR)**

(74) Mandataire: **Cabinet Plasseraud et al**
**235 Cours Lafayette**
**69006 Lyon (FR)**

(56) Documents cités:
**EP-A- 0 475 536     EP-A- 1 062 955**
**EP-A- 1 123 700**

**EP 1 492 531 B3**

**Description**

Le domaine de l'invention est celui de la libération modifiée de principes actifs pharmaceutiques. Dans le présent exposé, l'expression libération modifiée", désigne indifféremment une libération du (ou des) principe(s) actif(s) débutant dès la mise en contact de la forme galénique avec son milieu de dissolution (in vivo ou in vitro) ou bien encore une libération du (ou des) principe(s) actif(s) ne débutant qu'après une durée prédéterminée allant par exemple de 0,5 à plusieurs heures. Au sens de l'invention, le temps de libération de 50% du (ou des) principe(s) actif(s) est typiquement de plusieurs heures, et peut s'étendre de 0,5 à 30 heures, par exemple.

Plus précisément, l'invention concerne des formulations pharmaceutiques liquides, administrables oralement, à libération modifiée d'amoxicilline (antibiotique de la famille des β-lactames). Ces formulations sont constituées par des suspensions ou dispersions de microcapsules formées chacune par un coeur comprenant de l'amoxicilline et par un enrobage enveloppant ledit coeur. Les microcapsules constituant la phase dispersée de la suspension sont conçues, conformément à l'invention, pour permettre la libération modifiée de l'amoxicilline.

L'invention vise également des formulations pharmaceutiques sèches, destinées à être utilisées en suspensions aqueuses reconstituées au début du traitement. Ces suspensions aqueuses reconstituées sont stables pendant toute la durée du traitement et permettent la libération modifiée de l'amoxicilline.

Ces suspensions sont particulièrement intéressantes pour

- augmenter l'intervalle de temps entre deux prises, par exemple toutes les 12 heures au lieu de toutes les 8 heures;
- rendre plus aisée l'administration orale de médicament à base d'amoxicilline. En effet, un grand nombre de patients préfère boire une suspension médicamenteuse liquide, plutôt qu'avaler un ou plusieurs comprimés. L'observance s'en trouve ainsi améliorée. Cela intéresse notamment au plus haut point les populations incapables d'avaler des comprimés souvent de taille importante : les nourrissons, les enfants et les personnes âgées;
et ce, tout en améliorant le goût de la suspension.

L'invention vise également un procédé particulier de préparation des microcapsules d'amoxicilline destinées à être mises en suspension aqueuse.

On connaît de nombreux types de microcapsules sous forme sèche. En particulier, le brevet EP-B-0 709 087 décrit un système galénique (pharmaceutique ou diététique), de préférence sous forme de comprimé, avantageusement délitable, de poudre ou de gélule, caractérisé en ce qu'il comprend des microcapsules, de type réservoir, contenant au moins un Principe Actif médicamenteux et/ou nutritionnel (PA), notamment choisis parmi les antibiotiques, destinées à l'administration par voie orale, caractérisées :

- en ce qu'elles sont constituées par des particules de PA recouvertes chacune d'une pellicule d'enrobage de composition suivante :

    1- au moins un polymère filmogène (P1) insoluble dans les liquides du tractus, présent à raison de 50 à 90 % en poids sur sec par rapport à la masse totale de la composition d'enrobage et constitué par au moins un dérivé non hydrosoluble de la cellulose, l'éthylcellulose et/ou l'acétate de cellulose étant particulièrement préférés ;
    2- au moins un polymère azoté (P2) présent à raison de 2 à 25% en poids sur sec par rapport à la masse totale de la composition d'enrobage et constitué par au moins un polyacrylamide et/ou un poly-N-vinylamide et/ou un poly-Nvinyl-lactame, le polyacrylamide et/ou la polyvinylpyrrolidone étant particulièrement préférés ;
    3- au moins un plastifiant présent à raison de 2 à 20 % en poids sur sec par rapport à la masse totale de la composition d'enrobage et constitué par au moins l'un des composés suivants : les esters du glycérol, les phtalates, les citrates, les sébacates, les esters de l'alcool cétylique, l'huile de ricin, l'acide salicylique et la cutine, l'huile de ricin étant particulièrement préférée ;
    4- au moins un agent tensio-actif et/ou lubrifiant, présent à raison de 2 à 20 % en poids sur sec par rapport à la masse totale de la composition d'enrobage et choisi parmi les tensio-actifs anioniques, de préférence les sels alcalins ou alcalinoterreux des acides gras, l'acide stéarique et/ou oléique étant préférés, et/ou parmi les tensio-actifs non ioniques, de préférence les esters de sorbitan polyoxyéthylénés et/ou les dérivés de l'huile de ricin polyoxyéthylénés, et/ou parmi les agents lubrifiants comme les stéarates, de préférence de calcium, de magnésium, d'aluminium ou de zinc, ou comme le stéarylfumarate, de préférence de sodium, et/ou le béhénate de glycérol ; ledit agent pouvant comprendre un seul ou un mélange des susdits produits ;

- en ce qu'elles possèdent une granulométrie comprise entre 50 et 1 000 microns,
- en ce qu'elles sont conçues de manière à pouvoir séjourner dans l'intestin grêle pendant un temps d'au moins 5 heures environ, et permettre ainsi l'absorption du PA pendant au moins une partie de leur temps de séjour dans l'intestin grêle.

Ce document ne concerne que les formes pharmaceutiques sèches à base de microcapsules et ne mentionne nullement les formes pharmaceutiques liquides orales à base de microcapsules.

A ce jour, les formulations pharmaceutiques orales à libération modifiée d'amoxicilline n'existent que sous la forme de comprimés. Les brevets WO-A-94/27557, WO-A-95/20946, WO-A-95/28148, WO-A-96/04908, WO-A-00/61115, WO-A-00/61116, WO-A-01/47499, WO-A-02/30392 décrivent différentes formulations de tels comprimés. Ces comprimés sont de taille importante, comme par exemple le comprimé de l'exemple 1 du brevet WO 02/30392 qui présente une masse totale de 1600 mg pour une dose de 1000 g. Ces comprimés ne peuvent pas être administrés à des patients ayant des difficultés à avaler et encore moins à des enfants ou des nourrissons qui ne peuvent pas davantage les avaler et qui, de surcroît, nécessitent une adaptation de la dose administrée en fonction de leur poids. Pour de telles applications, les suspensions ou solutions multidoses sont, contrairement aux comprimés, des formes pharmaceutiques de choix.

Des suspensions ou solutions pharmaceutiques d'amoxicilline existent pour répondre à ce besoin. Ce sont par exemple les formulations décrites par les demandes de brevet WO-A-98/35672 ou WO-A-98/35672. Les efforts d'amélioration de ces suspensions ont porté sur la stabilité de la suspension (demande de brevet WO-A 00/50036) ou bien encore sur la tolérance gastro-intestinale (demandes de brevet WO-A-97/06798 & WO-A-00/03695) ou bien l'amélioration du goût (demande de brevet WO-A-98/36732).

Mais aucune de ces suspensions connues ne permet la libération modifiée de l'amoxicilline, nécessaire pour augmenter la durée d'action de l'amoxicilline et traiter certaines indications comme par exemple les pneumonies à streptocoques résistants. Pour répondre à ce besoin, une formulation contenant davantage d'amoxicilline par rapport au régime usuel est décrite dans la demande de brevet WO-A-97/09042, où l'amoxicilline se présente sous forme à libération immédiate.

La réalisation de suspensions liquides à libération modifiée d'amoxicilline est délicate. La difficulté principale à résoudre est d'éviter la libération de l'amoxicilline dans la phase liquide durant le stockage de la suspension, tout en permettant une libération modifiée dès son entrée dans le tractus gastro-intestinal. Cet objectif est particulièrement délicat à atteindre puisque l'amoxicilline est conservée dans un liquide pendant une très longue durée (la durée du traitement, soit une dizaine de jours) au regard du temps de libération souhaité dans les fluides du tractus gastro-intestinal (quelques heures, au plus 12 heures). Par ailleurs, son séjour prolongé dans la phase liquide de stockage ne doit pas perturber le système à libération modifiée au point d'entraîner une altération du profil et du temps de libération de l'amoxicilline.

Par ailleurs, pour que ces formulations liquides soient pleinement efficaces, on sait qu'il importe :

o que les microcapsules soient de très petite taille (< 1000 microns),
o et que la fraction massique en excipients d'enrobage soit limitée, cette modalité étant d'autant plus délicate à obtenir que, du fait de leur faible taille, les microcapsules ont une grande surface spécifique, ce qui accélère la libération.

S'agissant de l'art antérieur sur les formes pharmaceutiques liquides, orales, à libération modifiée de principes actifs, il convient de mentionner tout d'abord la demande de brevet PCT WO-A-87/07833 et le brevet US-B-4,902,513 divulguent des suspensions aqueuses de microcapsules de principe actif (e.g. théophylline), à libération modifiée du principe actif (12 h e.g.). Ces suspensions sont préparées en saturant la phase aqueuse avec le principe actif, avant d'incorporer les microcapsules de principe actif dans cette phase aqueuse. La composition de l'enrobant des microcapsules, permettant la libération modifiée du principe actif, n'est pas décrite dans ces documents. Or, cette composition d'enrobage est déterminante pour garantir le maintien du profil de libération modifiée des microcapsules, après conservation dans la phase aqueuse. Il apparaît que la proposition technique décrite ne divulgue pas les moyens permettant de résoudre le double problème de la réalisation d'une suspension liquide d'une forme microcapsulaire à libération modifiée, sans porter atteinte à la stabilité du profil de libération modifiée du principe actif à l'issue du stockage des microcapsules en phase liquide.

La demande de brevet européen EP-A-0 601 508 concerne une suspension aqueuse pour l'administration orale de naxoprène, selon un profil de libération modifiée. Cette suspension comprend des microgranules de naxoprène enrobés, en suspension dans une phase liquide aqueuse sirupeuse. Le problème technique sous-tendant cette invention est la fourniture d'une forme à libération modifiée de naxoprène, dosée à 1000 mg et administrable en une seule prise quotidienne.

Les microgranules sont constitués de naxoprène, de polyvinylpyrrolidone, et de lactose (90-300$\mu$m). Leur enrobage est fait de 4 couches. La première comprend: diéthylcellulose/diéthylphtalate/polyéthylèneglycol. La deuxième est à base de copolymères (meth)acrylatesl(meth)acryliques EUDRAGIT®. La troisième comporte du stéarate de glycérol/de la cire/des alcools gras. Et la quatrième est constituée par un revêtement entérique à base d'acétate/phtalate de cellulose. La libération de naxoprène intervient de manière modifiée sur 24 heures.

L'exemple 22 de cette demande de brevet européen EP-A-0601508 comporte une démonstration de la stabilité du profil de libération après 30 jours de stockage de la suspension liquide.

L'un des inconvénients de cette suspension résulte de la couche entérique qui interdit l'usage d'une suspension à pH neutre car cette couche est conçue pour se déliter et devenir liquide à ce pH neutre. Un autre inconvénient de cette couche entérique est qu'elle bloque la libération du principe actif dans l'estomac, à pH acide. Or , pour l'amoxicilline dont la fenêtre d'absorption est située dans les parties hautes du tractus gastrointestinal, il est primordial de la libérer dès l'arrivée dans l'estomac, pour qu'elle soit effectivement absorbée. Par ailleurs, il s'agit d'une solution multicouches très complexe et de surcroît spécifique au naproxène.

La demande de brevet PCT WO-A-96/01628 divulgue une formulation pharmaceutique liquide pour l'administration orale, selon un profil de libération modifiée (12 heures), d'un principe actif constitué par de la moguistéine. Le but visé est de proposer une formulation liquide de moguistéine à libération modifiée, qui soit facile à mesurer et à ingérer, qui ait une durée de libération permettant d'éviter les multiples prises, qui soit stable en suspension aqueuse dans le temps, qui soit savoureuse pour favoriser le respect de l'observance et dont la fabrication n'implique pas le recours à des matières toxiques telles que des solvants. Pour atteindre ce but, l'invention selon la demande de brevet PCT WO-A-96/01628 propose une suspension dans une phase liquide faiblement hydratée (essentiellement à base de sorbitol et de glycérine), de microgranules (90-300 $\mu$m) de moguistéine enrobés par une première couche hydrophile constituée d'acétate/phtatate de cellulose et de diéthylphtalate; par une seconde couche hydrophobe comportant du stéarate de glycérol/de la cire/des alcools gras; et par une troisième couche hydrophile identique à la première. Cette forme multicouche est très complexe à préparer et, de surcroît, est spécifique à la moguistéine.

EP-A-1 062955 décrit des microcapsules d'amoxicilline comprenant une double couche d'enrobage. Les microcapsules décrites peuvent être administrées en suspension.

WO-A-91 19486 décrit des microcapsules comprenant un enrobage similaire, mais pas identique à la présente demande, l'utilité de l'administration sous forme de suspension surtout pour des patients qui ont difficulté à avaler des comprimés ou capsules, et des exemples de microcapsules et de suspensions.

Dans cet état de la technique, la présente invention a pour objectif essentiel de proposer une suspension aqueuse ou une préparation pour suspension aqueuse, de microcapsules d'amoxicilline, pour l'administration orale d'amoxicilline selon un profil de libération modifiée, dans laquelle l'enrobage des microcapsules est conçu de telle sorte que le profil de libération n'est pas perturbé et ne dépend pas du temps de macération des microcapsules dans la phase liquide (de préférence aqueuse). Ainsi, serait évitée la libération de l'amoxicilline contenue dans les microcapsules, dans la phase liquide tout au long du stockage de la suspension, tout en permettant une libération modifiée de l'amoxicilline dès son entrée dans un environnement permettant le déclenchement de la libération, à savoir in vivo dans le tractus gastro-intestinal et in vitro dans les conditions d'un test de dissolution réalisé juste après la mise en suspension des microcapsules dans la phase solvant (de préférence aqueuse), à l'aide d'un appareil de type II, selon la pharmacopée européenne 3$^{\text{éme}}$ édition, dans un milieu tampon phosphate pH 6,8, pour un volume de 900 ml, à une température de 37°C. Un autre objectif de la présente invention est de fournir une suspension liquide aqueuse, de microcapsules d'amoxicilline comprenant une pellicule d'enrobage formée par une seule couche.

Un autre objectif de la présente invention est de fournir une suspension liquide aqueuse de microcapsules d'amoxicilline dans laquelle la fraction dissoute issue des microcapsules est inférieure ou égale à 15 %, de préférence à 5 % en poids de la masse totale d'amoxicilline présente dans les microcapsules.

Un autre objectif de la présente invention est de fournir une suspension liquide aqueuse de microcapsules d'amoxicilline dans laquelle une partie de l'amoxicilline est sous forme à libération immédiate et l'autre partie de l'amoxicilline est sous forme à libération modifiée (microcapsules).

Un autre objectif essentiel de la présente invention est de fournir une suspension aqueuse de microcapsules à libération modifiée d'amoxicilline permettant de libérer l'amoxicilline selon un profil de libération qui n'est pas altéré par le temps de vieillissement de la suspension.

Un autre objectif essentiel de la présente invention est de fournir une suspension aqueuse de microcapsules faite de particules d'amoxicilline enrobées individuellement, et permettant la libération de cette dernière selon un profil prolongé et/ou éventuellement retardé, tel que le temps de demi-libération $t_{1/2}$ soit compris entre 0,5 et 30 heures.

Un autre objectif de la présente invention est de proposer une forme galénique orale qui soit liquide et constituée d'un grand nombre (par exemple de l'ordre de plusieurs milliers) de microcapsules, cette multiplicité assurant statistiquement une bonne reproductibilité de la cinétique de transit du PA dans tout le tractus gastro-intestinal, de sorte qu'il en résulte un meilleur contrôle de la biodisponibilité et donc une meilleure efficacité.

Un objectif essentiel de la présente invention est de proposer une forme galénique liquide, orale et formée d'une pluralité de microcapsules enrobées évitant l'emploi de fortes quantités d'enrobant, la fraction massique d'enrobant étant comparable à celle des formes monolithiques.

Un autre objectif essentiel de la présente invention est de fournir une suspension aqueuse à libération modifiée dans laquelle l'amoxicilline est sous forme d'une pluralité de particules enrobées individuellement pour former des microcapsules et permettant le mélange avec d'autres principes actifs, libérés selon des temps de libération respectifs différents.

Un autre objectif essentiel de la présente invention est de proposer l'utilisation, à titre de moyen de traitement de maladies humaines ou vétérinaires, d'une suspension (de préférence aqueuse) de microcapsules constituées par des particules d'amoxicilline enrobées individuellement de façon à déterminer la libération modifiée de l'amoxicilline, sans que le stockage sous cette forme liquide des microcapsules dans la suspension n'ait d'incidence sur le profil de libération modifiée.

Un autre objectif essentiel de la présente invention est de proposer un médicament à base d'une suspension de préférence aqueuse de microcapsules constituées par des particules d'amoxiciline enrobées individuellement de façon à déterminer la libération modifiée de l'amoxicilline, sans que le stockage sous cette forme liquide des microcapsules dans la suspension n'ait d'incidence sur le profil de libération modifiée.

S'étant fixé tous les objectifs ci-dessus, parmi d'autres, les inventeurs ont eu le mérite de mettre au point un système galénique multimicrocapsulaire sous forme de suspension, de préférence aqueuse, à libération modifiée d'amoxicilline :

- qui n'altère pas le profil de libération modifiée, éventuellement retardée,
- et qui soit stable, facile à préparer, économique et performante.

Pour ce faire, les inventeurs ont proposé :

- de sélectionner une composition d'enrobage tout à fait particulière pour les microcapsules,
- et de mettre les microcapsules en suspension dans une phase liquide (de préférence aqueuse) saturée en amoxicilline ou saturable en amoxicilline au contact des microcapsules, tout en mettant en oeuvre une quantité limitée de solvant (de préférence l'eau), mais néanmoins suffisante pour que la suspension puisse être aisément avalée.

Ainsi, l'invention qui satisfait aux objectifs exposés ci-dessus, parmi d'autres, concerne une suspension de microcapsules dans une phase liquide aqueuse, ladite suspension étant destinée à être administrée par voie orale et permettant la libération modifiée d'amoxicilline, caractérisée :

■ en ce qu'elle comprend une pluralité de microcapsules constituées chacune par un coeur contenant l'amoxicilline et par une pellicule d'enrobage :

- • appliquée sur le coeur,
- • régissant la libération modifiée de l'amoxicilline,
- • et ayant une composition correspondant à l'une des familles A et C suivantes :

⇒ Famille A

♦ 1A -au moins un polymère filmogène (P1) insoluble dans les liquides du tractus, présent à raison de 50 à 90, de préférence 50 à 80 en poids sur sec par rapport à la masse totale de la composition d'enrobage et constitué par au moins un dérivé non hydrosoluble de la cellulose,

♦ 2A -au moins un polymère azoté (P2) présent à raison de 2 à 25, de préférence 5 à 15 % en poids sur sec par rapport à la masse totale de la composition d'enrobage et constitué par au moins un polyacrylamide et/ou un poly-N-vinylamide et/ou un polyN-vinyl-lactanie;

♦ 3A -au moins un plastifiant présent à raison de 2 à 20, de préférence de 4 à 15 % en poids sur sec par rapport à ta masse totale de la composition d'enrobage et constitué par au moins l'un des composés suivants : les esters du glycérol, les phtalates, les citrates, les sébaçates, les esters de l'alcool cétylique, l'huile de ricin;

♦ 4A -au moins un agent tensio-actif et/ou lubrifiant, présent à raison de 2 à 20, de préférence de 4 à 15 % en poids sur sec par rapport à la masse totale de la composition d'enrobage et choisi parmi les tensio-actifs anioniques, et/ou parmi les tensio-actifs non ioniques, et/ou parmi les agents lubrifiants; ledit agent pouvant comprendre un seul ou un mélange des susdits produits;

⇒ Famille C :

♦ 1C -au moins un polymère filmogène insoluble dans les liquides du tractus gastro-intestinal,

♦ 2C -au moins un polymère hydrosoluble,

♦ 3C -au moins un plastifiant,

♦ 4C -et éventuellement au moins un agent tensioactif/lubrifiant de préférence sélectionné dans le groupe de produits suivants:

~ les tensioactifs anioniques,
~ et/ou les tensioactifs non ioniques:

■ et en ce que la phase liquide est saturée ou se sature en amoxicilline au contact des microcapsules.

Au sens du présent exposé le terme *"microcapsules d'amoxicilline"* désigne des microcapsules dont le coeur comprend de l'amoxicilline et éventuellement au moins un autre principe actif et/ou au moins un excipient

Cette suspension selon invention permet de surmonter les deux principaux obstacles à la réalisation d'une suspension aqueuse de microcapsules constituées par des microparticules d'amoxiciline enrobées individuellement et permettant la libération modifiée de cette dernière, ces deux obstacles étant de :

a) limiter la fraction d'amoxicilline libérable immédiatement à partir des microcapsules, à une valeur inférieure à 15 %, et de préférence à 5 % en poids de la masse totale d'amoxicilline mise en oeuvre dans les microcapsules,
b) obtenir un système à libération modifiée suffisamment robuste pour éviter toute évolution ou altération du profil de libération de l'amoxicilline durant la durée de stockage de la suspension aqueuse.

Selon une forme de réalisation préférée de l'invention, les familles A et C dans lesquelles sont choisies les constituants de la composition d'enrobage sont les suivantes :

⇒ Famille A

♦ 1A - éthylcellulose et/ou acétate de cellulose;
* 2A - polyacrylamide et/ou polyvinyl-pyrrolidone;
♦ 3A - huile de ricin;
♦ 4A - sel alcalin ou alcalinoterreux des acides gras, l'acide stéarique et/ou oléique étant préférés, ester de sorbitan polyoxyéthyléné dérivé de l'huile de ricin polyoxyéthyléné, stéarate, de préférence de calcium, de magnésium, d'aluminium ou de zinc, stéarylfumarate, de préférence de sodium béhénate de glycérol ; pris à eux seuls ou en mélange entre eux;

⇒ Famille C :

♦ 1C

~ les dérivés non hydrosolubles de la cellulose, l'éthylcellulose et/ou l'acétate de cellulose étant particulièrement préférés,
~ les dérivés acryliques,
~ les polyvinylacétates,
~ et leurs mélanges.

♦ 2C

- les dérivés hydrosolubles de la cellulose,
- les polyacrylamides,
- les poly-N-vinylamides,
- les poly-N-vinyl-lactames,
- les alcools polyvinyliques (APV),
- les polyoxyéthylènes (POE).
- les polyvinylpyrrolidones (PVP) (ces dernières étant préférées),
- et leurs mélanges ;

♦ 3C

- le glycérol et ses esters, de préférence dans le sous-groupe suivant: glycérides acétylés, glycérolmonostéarate, glycéryltriacétate, glycéroltributyrate,
- les phtalates, de préférence dans le sous-groupe suivant : dibutylphthalate, diéthylphthalate, diméthylphthalate, dioctylphthalate,

- les citrates, de préférence dans le sous-groupe suivant : acétyltributylcitrate, acétyltriéthylcitrate, tributylcitrate, triéthylcitrate,
- les sébacates, de préférence dans le sous-groupe suivant : diéthylsébaçate, dibutylsébaçate,
- les adipates,
- les azélates,
~ les benzoates,
~ les huiles végétales,
- les fumarates de préférence le diéthylfumarate,
- les malates, de préférence le diéthylmalate,
~ les oxalates, de préférence le diéthyloxalate,
- les succinates; de préférence le dibutylsuccinate,
- les butyrates,
- les esters de l'alcool cétylique,
~ l'acide salicylique,
- la triacétine,
- les malonates, de préférence le diéthylmalonate,
- la cutine,
- l'huile de ricin (celle-ci étant particulièrement préférée),
- et leurs mélanges;

♦ 4C

- les sels alcalins ou alcalinoterreux des acides gras, l'acide stéarique et/ou oléique étant préférés,
- les huiles polyoxyéthylénées de préférence l'huile de ricin hydrogénée polyoxyéthylénée.
les copolymères polyoxyéthylène-polyoxypropylène,
les esters de sorbitan polyoxyéthylénés,
les dérivés de l'huile de ricin polyoxyéthylénés,
les stéarates, de préférence de calcium, de magnésium, d'aluminium ou de zinc,
les stéarylfumarates, de préférence de sodium,
le béhénate de glycérol,
et leurs mélanges.

Selon une modalité avantageuse de l'invention et lorsque l'enrobage contient de la cire, cette dernière est sélectionnée parmi les composés ayant une température de fusion $T_f \geq 40°C$, de préférence $T_f \geq 50°C$.

De préférence, la pellicule d'enrobage est constituée par une seule couche, dont la masse représente de 1 à 50 % en poids, de préférence de 5 à 40 % en poids, de la masse totale des microcapsules.

Suivant une caractéristique préférée de l'invention, la phase liquide est aqueuse, et plus préférentiellement encore, elle est constituée d'au moins 20% d'eau, et mieux encore d'au moins 50 % en poids d'eau.

Cette suspension selon l'invention comporte, avantageusement :

- 30 à 95 % en poids, de préférence 60 à 85 % en poids de phase liquide (avantageusement d'eau),
- 5 à 70 % en poids de préférence 15 à 40 % en poids de microcapsules.

Avantageusement, la quantité de phase liquide solvant (de préférence l'eau) de l'amoxicilline est telle que la proportion d'amoxicilline dissoute et provenant des microcapsules soit inférieure ou égale à 15%, de préférence à 5 % en poids par rapport à la masse totale d'amoxicilline contenue dans les microcapsules.

Selon un premier mode de réalisation de l'invention, la phase liquide est au moins en partie, de préférence totalement, saturée en amoxicilline consécutivement à l'incorporation des microcapsules dans cette phase liquide. Selon ce mode de réalisation, la saturation en amoxicilline s'opère au moyen de l'amoxicilline contenue dans les microcapsules.

Selon un deuxième mode de réalisation de l'invention, la phase liquide est au moins en partie, de préférence totalement, saturée en amoxicilline à l'aide d'amoxicilline non encapsulée, avant l'incorporation des microcapsules dans cette phase liquide. Ce mode de réalisation est particulièrement intéressant pour l'administration d'amoxicilline, en ce qu'il permet d'associer une fraction à libération immédiate et une fraction à libération modifiée.

En pratique, cela revient à saturer la phase liquide en amoxicilline avant l'introduction des microcapsules dans la suspension, de sorte que l'amoxicilline contenue dans les microcapsules ne participe pas, ou quasiment pas, à la saturation de la phase liquide. La diffusion de l'amoxicilline contenue dans les microcapsules est donc supprimée ou quasiment supprimée.

Suivant une caractéristique préférée de l'invention permettant à cette formulation orale liquide d'être pleinement efficace, les microcapsules ont une granulométrie comprise inférieure ou égale à 1.000 microns, de préférence comprise entre 200 et 800 microns, et, plus préférentiellement encore, entre 200 et 600 microns.

Par « granulométrie », on entend au sens de l'invention, une proportion d'au moins 75 % en poids de microcapsules de diamètre compris entre les limites considérées de taille de grille de tamisage.

Toujours dans le souci d'améliorer l'efficacité, la quantité d'enrobant des microcapsules représente avantageusement de 1 à 50%, de préférence 5 à 40 %, du poids des microcapsules enrobées. Cette caractéristique avantageuse est d'autant à acquérir que les microcapsules, du fait de leur faible taille, ont une grande surface spécifique, ce qui accélère la libération.

Pour contrôler la libération in vivo in vitro de l'amoxicilline, il est préférable, conformément à l'invention, de mettre en oeuvre une pellicule d'enrobage pour les microcapsules, ayant une composition relevant de la famille A ou C.

Pour plus de données détaillées sur le plan qualitatif et quantitatif, pour ce qui concerne cette composition d'enrobage de famille A, on se référera au brevet européen EP-B-0 709 087 dont le contenu est intégré dans le présent exposé par référence.

Un autre moyen de définition de la suspension liquide selon l'invention peut consister à faire état d'un profil de libération in vitro réalisé à l'aide d'un appareil de type II, selon la pharmacopée européenne 3ème édition, dans un milieu tampon phosphate pH 6.8, et à une température de 37°C, tel que:

► la proportion PI d'amoxicilline libérée à partir des microcapsules durant les 15 premières minutes du test de dissolution est telle que :

$$PI \leq 15$$

de préférence $PI \leq 5$.

► la libération de l'amoxicilline restant dans les microcapsules s'effectue sur une durée telle que le temps de libération de 50 % en poids de l'amoxicilline ($t_{1/2}$) se définit comme suit (en heures) :

$$0,5 \quad \leq t_{1/2} \leq 30$$

de préférence

$$0,5 \quad \leq t_{1/2} \leq 20.$$

Toujours en ce qui concerne ses propriétés de dissolution in vitro, la suspension selon l'invention est caractérisée en ce que :

- le profil de libération in vitro initial Pfi, réalisé juste après la mise en suspension des microcapsules dans la phase solvant (de préférence aqueuse), à l'aide d'un appareil de type II, selon la pharmacopée européenne 3éme édition, dans un milieu tampon phosphate pH 6,8, pour un volume de 900 ml, à une température de 37°C,
- et le profil de libération in vitro $Pf_{10}$, réalisé 10 jours après la mise en suspension des microcapsules dans la phase solvant (de préférence aqueuse), à l'aide d'un appareil de type II, selon la pharmacopée européenne 3ème édition, dans un milieu tampon phosphate pH 6,8, à une température de 37°C, sont similaires.

Les profils de libération comparés selon les recommandations de l'Agence Européenne pour l'Evaluation des produits médicinaux -Unité d'évaluation des médicaments humains- *"The European Agency for the Evaluation of Medicinal Products (EMEA)- Human Medicines Evaluation Unit- / Committee for proprietary medicinal products (CPMP) - London, 29 july 1999 CPMP/QWP/604/96 : note for guidance on quality of modified release products : A :oral dosage forms B : transdermal dosage forms -section I (quality)- Annex 3 : Similarity factor $f_2$"*, conduisent à une valeur des facteurs de similarité $f_2$ >50 et peuvent donc être déclarés similaires.

Ces caractéristiques avantageuses de la suspension selon l'invention permettent d'administrer oralement, de façon aisée, l'amoxicilline sans nuire au mode de libération modifiée et éventuellement retardée.

Suivant une autre de ses caractéristiques physico-chimiques avantageuses, le pH de la suspension liquide selon l'invention peut être indifféremment acide ou neutre.

Il peut être tout à fait intéressant d'adjoindre à la suspension au moins un agent modificateur de la rhéologie. Il peut s'agir en particulier, d'un ou plusieurs agents "viscosifiants" choisis ceux communément employés dans l'industrie pharmaceutique et notamment ceux divulgués dans *"*Handbook of pharmaceutical excipients - 3 rd Edition, Am. Pharmaceutical Association, Arthur H KIBBE, 2000, ISBN 0917330-96-X. Europe. 0-85369-381-1*"*. A titre d'exemples, on peut citer :

- les dérivés hydrosolubles de la cellulose (hydroxyethyl cellulose, hydroxypropyl cellulose, carboxymethyl cellulose, hydroxypropylmethyl cellulose ...) ;
- les polyethylene glycol ;
- les alginates et leurs dérivés;
- les carraghénanes ;
- l'agar-agar ;
- la gélatine;
- les maltodextrines ;
- le polydextrose ;
- les gommes guar, caroube, acacia, xanthane, gellane, ...
- l'alcool polyvinylique ;
- la povidone ;
- les pectines ;
- le gel de silice ;
- les amidons natifs, modifiés et leurs dérivés ;
- les dextrans.
- etc ...

Il peut être également judicieux d'introduire dans la suspension au moins un agent modificateur de la solubilité de l'amoxicilline dans la phase liquide-solvant (de préférence aqueuse), comme par exemple des sels, des sucres, du glycérol, ....

Pour donner à la suspension toutes les qualités d'une forme galénique orale, facile à avaler, stable et appétente, il est avantageux qu'elle comprenne au moins un autre additif choisi dans le groupe comprenant : les tensioactifs, les colorants, les agents dispersants, les conservateurs, les agents de sapidité, les arômes, les édulcorants, les anti-oxydants et leurs mélanges.

A titre d'exemples de ces additifs, on peut citer ceux communément employés dans l'industrie pharmaceutique et notamment ceux divulgués dans *"*Handbook of pharmaceutical excipients - 3 rd Edition, Am. Pharmaceutical Association, Arthur H KIBBE, 2000, ISBN 0917330-96-X. Europe. 0- 85369-381-1*"* ou s'agissant des émulsifiants, ceux décrits à la page 5, ligne 14 à 29 de l'EP-A- 0 273 890, ou bien encore s'agissant des épaississants, ceux indiqués à la page 5 lignes 19 et 20 de l'EP-A-0 601 508.

Selon un autre de ses aspects, la présente invention concerne un médicament caractérisé en ce qu'il comprend de la suspension de microcapsules d'amoxicilline à libération modifiée, telle que définie ci-dessus.

Plus concrètement, l'invention vise également un médicament, ou plus exactement un conditionnement galénique, caractérisé en ce qu'il comprend un nécessaire de préparation de la suspension telle que définie ci-dessus, lequel nécessaire comportant :

- des microcapsules sous forme sensiblement sèche contenant l'amoxicilline pour permettre la saturation de la phase liquide en amoxicilline, une fois réalisée la mise en présence des deux phases solide et liquide;
- et/ou un mélange de microcapsules sous forme sensiblement sèche comprenant la dose en amoxicilline juste nécessaire pour la libération modifiée ainsi qu'une quantité nécessaire et suffisante d'amoxicilline non enrobée à libération immédiate, pour permettre la saturation de la phase liquide en amoxicilline une fois réalisée la mise en présence de la dose de saturation d'amoxicilline et de la phase liquide;
- et la phase liquide et/ou au moins une partie des ingrédients utiles à sa préparation et/ou le protocole de préparation de la suspension.

Ce type de présentation du médicament selon l'invention, permet aux patients de préparer aisément la suspension à libération modifiée, sous une forme stable, en particulier en termes de libération modifiée, et ce pendant au moins plusieurs jours. Le patient est donc ainsi garanti de disposer d'un médicament facilement administrable oralement, et parfaitement efficace sur le plan thérapeutique.

S'agissant de la préparation des microcapsules constituant la phase solide de la suspension selon l'invention, cela renvoie à des techniques de microencapsulation accessibles à l'homme du métier, dont les principales sont résumées dans l'article de C. DUVERNEY et J.P. BENOIT dans "L'actualité chimique", décembre 1986. Plus précisément, la technique considérée est la microencapsulation par pelliculage, conduisant à des systèmes "réservoir" individualisés par opposition aux systèmes matriciels.

Pour plus de détails, on se référera au brevet EP-B-0 953 359 mentionné ci-avant.

Pour la réalisation du coeur à base d'amoxicilline des microcapsules selon l'invention, il est avantageux d'utiliser comme matières premières, des particules d'amoxicilline de granulométrie désirée. Ces dernières peuvent être des cristaux d'amoxicilline purs et/ou ayant subi un prétraitement par l'une des techniques conventionnelles en la matière, comme par exemple la granulation, en présence d'une faible quantité d'au moins un agent liant classique et/ou d'un agent modifiant les caractéristiques de solubilité intrinsèque de l'amoxicilline.

L'invention sera mieux comprise sur le plan de sa composition de ses propriétés et de son obtention, à la lecture des exemples ci-après, donnés uniquement à titre d'illustration et permettant de faire ressortir les variantes de réalisation et les avantages de l'invention.

Description des figures :

- **La figure 1** montre les profils de dissolution initial et après 12 jours de conservation de la suspension selon l'exemple 1, en % dissous en fonction du temps en heures.
- **La figure 2** montre les profils de dissolution initial et après 12 jours de conservation de la suspension selon l'exemple 2, en % dissous en fonction du temps en heures

Exemple 1

*Préparation de microcapsules d'amoxicilline:*

970 g d'amoxicilline trihydrate et 30 g de Povidone (Plasdone® K29/32) ) sont préalablement mélangés à sec dans la cuve d'un granulateur à haut cisaillement (Lödige® M5MRK) pendant 5 minutes. Ce mélange pulvérulent est ensuite granulé à l'eau (200 g). Les granulés sont séchés à 40°C en étuve ventilée, puis calibrés sur grille de 500 µm. On sélectionne par tamisage la fraction 200-500 µm.

700 g de granulés obtenus précédemment sont enrobés dans un appareil à lit d'air fluidisé GLATT GPCG1 par 107,6 g d'éthylcellulose (Ethocel 7 Premium), 35,3 g de povidone (Plasdone® K29/32) et 10,8 g d'huile de ricin dissous dans un mélange acétone / isopropanol (60/40 m/m)

*Préparation de la suspension:*

12,2 g de microcapsules obtenues ci-dessus sont mélangées à sec avec 0,3 g de gomme xanthane dans un flacon en verre de 100 ml. 87,5 g d'eau purifiée sont ensuite ajoutée sur le mélange de poudre. Après agitation manuelle, une suspension qui sédimente très lentement est obtenue.

Le titre en Amoxicilline dans la suspension est de 0,1 g/ml.

*Test de stabilisé:*

La suspension préparée ci dessus est conservée pendant 12 jours à température ambiante. Au bout de 12 jours, la suspension est analysée en dissolution. Appareil de type II selon la pharmacopée Européenne 3è édition, milieu tampon phosphate pH 6,8, volume 900 ml, température 37°C, agitation palettes 100 tours/min, détection UV 272 nm.

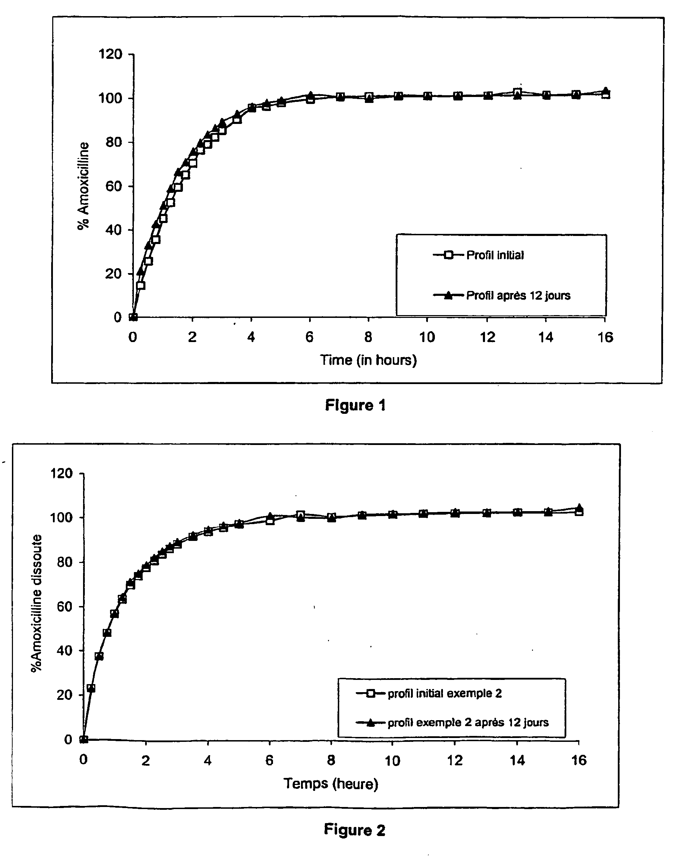

Le résultat est représenté sur la figure 1 annexée.

Il apparaît que les profils sont identiques : facteur de similarité $f_2$ supérieur à 50. Les microcapsules restent bien performantes en suspension aqueuse.

*Test d'homogénéité :*

La suspension ci-dessus est agitée manuellement puis à l'aide d'une seringue graduée, 6 échantillons de 5 ml sont prélevés. La teneur en Amoxicilline de chaque prélèvement est déterminée par HPLC et reportée ci-dessous :

| Prélèvement n° | Teneur en Amoxicilline pour 5 ml de suspension (en g) |
|---|---|
| 1 | 0,51 |

(suite)

| Prélèvement n° | Teneur en Amoxicilline pour 5 ml de suspension (en g) |
|---|---|
| 2 | 0,49 |
| 3 | 0,52 |
| 4 | 0,50 |
| 5 | 0,50 |
| 6 | 0,51 |

On constate que les prélèvements sont bien homogènes et que le dosage correspond à la valeur attendue de 0.5 g pour 5 ml.

Cette formulation peut donc être administrée sans risque de sur- ou sous-dosage.

Exemple 2 :

*Préparation de microcapsules d'amoxicilline:*

970 g d'amoxicilline trihydrate et 30 g de Povidone (Plasdone® K29/32) ) sont préalablement mélangés à sec dans la cuve d'un granulateur à haut cisaillement (ladite® M5MRK) pendant 5 minutes. Ce mélange pulvérulent est ensuite granulé à l'eau (200 g). Les granulés sont séchés à 40°C en étuve ventilée, puis calibrés sur grille de 500 $\mu$m. On sélectionne par tamisage la fraction 200-500 $\mu$m.

920 g de granulés obtenus précédemment sont enrobés dans un appareil à lit d'air fluidisé GLATT GPCG1 par 61g d'Aquacoat ECD30, 2,6g de povidone (Plasdone® K29/32) et 16,4 g de triéthylcitrate dispersés dans de l'eau.

*Préparation de la suspension:*

10,9 g de microcapsules obtenues ci-dessus sont mélangées à sec avec 0,3 g de gomme xanthane dans un flacon en verre de 100 ml. 88.8 g d'eau purifiée sont ensuite ajoutée sur le mélange de poudre. Après agitation manuelle, une suspension qui sédimente très lentement est obtenue.

Le titre en Amoxicilline dans la suspension est de 0,1 g/ml.

*Test de stabilité:*

La suspension préparée ci dessus est conservée pendant 12 jours à température ambiante. Au bout de 12 jours, la suspension est analysée en dissolution. Appareil de type II selon la pharmacopée Européenne 3è édition, milieu tampon phosphate pH 6,8, volume 900 ml, température 37°C, agitation palettes 100 tours/min, détection UV 272 nm.

Le résultat est représenté sur la figure 2 annexée.

Il apparaît que les profils sont identiques : facteur de similarité $f_2$ supérieur à 50. Les microcapsules restent bien performantes en suspension aqueuse.

**Revendications**

1.  Suspension de microcapsules dans une phase liquide aqueuse, ladite suspension étant destinée à être administrée par voie orale et permettant la libération modifiée d'amoxicilline, **caractérisée** :

    ■ en ce qu'elle comprend une pluralité de microcapsules constituées chacune par un coeur contenant l'amoxicilline et par une pellicule d'enrobage:

    • appliquée sur le coeur,
    • régissant la libération modifiée de l'amoxicilline,
    • et ayant une composition correspondant à l'une des familles A, ou C suivantes :

    ⇒ Famille A

♦ 1A -au moins un polymère filmogène (P1) insoluble dans les liquides du tractus, présent à raison de 50 à 90 % de préférence 50 à 80 % en poids sur sec par rapport à la masse totale de la composition d'enrobage et constitué par au moins un dérivé non hydrosoluble de la cellulose;

♦ 2A -au moins un polymère azoté (P2) présent à raison de 2 à 25 % de préférence 5 à 15 % en poids sur sec par rapport à la masse totale de la composition d'enrobage et constitué par au moins un polyacrylamide et/ou un poly-N-vinylamide et/ou un polyN-vinyllactame;

♦ 3A -au moins un plastifiant présent à raison de 2 à 20 % de préférence de 4 à 15 % en poids sur sec par rapport à la masse totale de la composition d'enrobage et constitué par au moins l'un des composés suivants : les esters du glycérol, les phtalates, les citrates, les sébacates, les esters de l'alcool cétylique, l'huile de ricin;

♦ 4A -au moins un agent tensio-actif et/ou lubrifiant, présent à raison de 2 à 20% de préférence de 4 à 15% en poids sur sec par rapport à la masse totale de la composition d'enrobage et choisi parmi les tensio-actifs anioniques, et/ou parmi les tensio-actifs non ioniques, et/ou parmi les agents lubrifiants; ledit agent pouvant comprendre un seul ou un mélange des susdits produits;

⇒ Famille C :

♦ 1C -au moins un polymère filmogène insoluble dans les liquides du tractus gastro-intestinal,
♦ 2C -au moins un polymère hydrosoluble,
♦ 3C -au moins un plastifiant,
♦ 4C -et éventuellement au moins un agent tensioactif/lubrifiant de préférence sélectionné dans le groupe de produits suivants:

- les tensioactifs anioniques,
- et/ou les tensioactifs non ioniques;

■ et en ce que la phase liquide est saturée ou se sature en amoxicilline au contact des microcapsules.

2. Suspension selon la revendication 1, **caractérisée en ce que** les familles A, et C de composition d'enrobage sont les suivantes :

⇒ Famille A :

♦ 1A - éthylcellulose et/ou acétate de cellulose;
♦ 2A - polyacrylamide et/ou polyvinyl-pyrrolidone;
♦ 3A - huile de ricin;
♦ 4A - sel alcalin ou alcalinoterreux des acides gras, l'acide stéarique et/ou oléique étant préférés, ester de sorbitan polyoxyéthyléné dérivé de l'huile de ricin polyoxyéthyléné, stéarate, de préférence de calcium, de magnésium, d'aluminium ou de zinc, stéarylfumarate, de préférence de sodium béhénate de glycérol ; pris à eux seuls ou en mélange entre eux;

⇒ Famille C :

♦ 1C

- les dérivés non hydrosolubles de la cellulose, l'éthylcellulose et/ou l'acétate de cellulose étant particulièrement préféré(s),
- les dérivés acryliques,
- les polyvinylacétates,
- et leurs mélanges.

♦ 2C

~ les dérivés hydrosolubles de la cellulose,
~ les polyacrylamides,
~ les poly-N-vinylamides,
~ les poly-N-vinyl-lactames,
~ les alcools polyvinyliques (APV),

~ les polyoxyéthylènes (POE),
~ les polyvinylpyrrolidones (PVP) (ces dernières étant préférées),
~ et leurs mélanges ;

♦ 3C

- le glycérol et ses esters, de préférence dans le sous-groupe suivant: glycérides acétylés, glycérolmonostéarate, glycéryltriacétate, glycéroltributyrate,
- les phtalates, de préférence dans le sous-groupe suivant : dibutylphtalate, diéthylphtalate, diméthylphtalate, dioctylphtalate,
- les citrates, de préférence dans le sous-groupe suivant : acétyltributylcitrate, acétyltriéthylcitrate, tributylcitrate, triéthylcitrate,
- les sébacates, de préférence dans le sous-groupe suivant : diéthylsébacate, dibutylsébacate,
- les adipates,
- les azélates,
- les benzoates,
- les huiles végétales,
- les fumarates de préférence le diéthylfumarate,
- les malates, de préférence le diéthylmalate,
- les oxalates, de préférence le diéthyloxalate,
- les succinates; de préférence le dibutylsuccinate,
- les butyrates,
- les esters de l'alcool cétylique,
- l'acide salicylique,
- la triacétine,
- les malonates, de préférence le diéthylmalonate,
- la cutine,
- l'huile de ricin (celle-ci étant particulièrement préférée),
- et leurs mélanges;

♦ 4C

- les sels alcalins ou alcalinoterreux des acides gras, l'acide stéarique et/ou oléique étant préféré(s),
- les huiles polyoxyéthylénées de préférence l'huile de ricin hydrogénée polyoxyéthylénée,
- les copolymères polyoxyéthylène-polyoxypropylène,
- les esters de sorbitan polyoxyéthylénés,
- les dérivés de l'huile de ricin polyoxyéthylénés,
- les stéarates, de préférence de calcium, de magnésium, d'aluminium ou de zinc,
- les stéarylfumarates, de préférence de sodium,
- le béhénate de glycérol,
- et leurs mélanges.

**3.** Suspension selon la revendication 1 ou 2, **caractérisée en ce que** la pellicule d'enrobage est constituée par une seule couche.

**4.** Suspension selon la revendication 1, **caractérisée en ce qu'**elle comporte :

- 30 à 95 % en poids, de préférence 60 à 85 % en poids de phase liquide (avantageusement d'eau),
- 5 à 70 % en poids de préférence 15 à 40 % en poids de microcapsules ;

**5.** Suspension selon la revendication 1, **caractérisée en ce que** la quantité de phase liquide solvant (de préférence l'eau) de l'amoxicilline est telle la proportion d'amoxicilline dissoute et provenant des microcapsules soit inférieure ou égale à 15 %, de préférence à 5 % en poids par rapport à la masse totale d'amoxicilline contenue dans les microcapsules.

**6.** Suspension selon la revendication 1, **caractérisée en ce que** la phase liquide est au moins en partie, de préférence totalement, saturée en amoxicilline consécutivement à l'incorporation des microcapsules dans cette phase liquide.

**7.** Suspension selon la revendication 6 **caractérisée en ce que** la saturation en amoxicilline s'opère au moyen du principe(s) actif(s) contenu(s) dans les microcapsules.

**8.** Suspension selon la revendication 1, **caractérisée en ce que** la phase liquide est au moins en partie, de préférence totalement, saturée en amoxicilline à l'aide d'amoxicilline non encapsulée, avant l'incorporation des microcapsules dans cette phase liquide.

**9.** Suspension selon l'une quelconque des revendications 1 à 8 **caractérisée en ce que** les microcapsules ont une granulométrie inférieure ou égale à 1.000 microns, de préférence comprise entre 200 et 800 microns, et, plus préférentiellement encore, entre 200 et 600 microns.

**10.** Suspension selon l'une quelconque des revendications 1 à 9, **caractérisée en ce que** la pellicule d'enrobage représente de 1 à 50 % en poids, de préférence de 5 à 40 % en poids, de la masse totale des microcapsules enrobées.

**11.** Suspension selon l'une quelconque des revendications 1 à 10, **caractérisée en ce que** les constituants de ladite suspension sont prévus en quantité telle que la suspension a un profil de libération in vitro réalisé à l'aide d'un appareil de type II, selon la pharmacopée européenne 3ème édition, dans un milieu tampon phosphate pH 6,8 et à une température de 37°C, tel que :

▶ la proportion (%) d'amoxicilline libérée durant les 15 premières minutes du test de dissolution est telle que:

$$PI \leq 15$$

de préférence

$$PI \leq 5,$$

▶ la libération de l'amoxicilline restante s'effectue sur une durée telle que le temps de libération de 50 % poids de PA ($t_{1/2}$) se définit comme suit (en heures) :

$$0,5 \leq t_{1/2} \leq 30$$

de préférence

$$0,5 \leq t_{1/2} \leq 20.$$

**12.** Suspension selon l'une quelconque des revendications 1 à 11, **caractérisée en ce que** les constituants de ladite suspension sont prévus en quantité telle que :

- le profil de libération in vitro initial Pfi, réalisé juste après la mise en suspension des microcapsules dans la phase solvant (de préférence aqueuse), à l'aide d'un appareil de type II, selon la pharmacopée européenne 3éme édition, dans un milieu tampon phosphate pH 6,8, à une température de 37°C,
- et le profil de libération in vitro Pf10, réalisé 10 jours après la mise en suspension des microcapsules dans la phase solvant (de préférence aqueuse), à l'aide d'un appareil de type II, selon la pharmacopée européenne 3ème édition, dans un milieu tampon phosphate pH 6,8, à une température de 37°C,

sont similaires.

**13.** Suspension selon l'une quelconque des revendications 1 à 12, **caractérisée en ce que** son pH est indifféremment acide ou neutre.

**14.** Suspension selon l'une quelconque des revendications 1 à 13, **caractérisée en ce qu'**elle comprend au moins un agent modificateur de la rhéologie.

**15.** Suspension selon l'une quelconque des revendications 1 à 14, **caractérisée en ce qu'**elle comprend au moins un agent modificateur de la solubilité de l'amoxicilline dans la phase liquide-solvant (de préférence aqueuse).

**16.** Suspension selon l'une quelconque des revendications 1 à 15, **caractérisée en ce qu'**elle comprend au moins un additif choisi dans le groupe comprenant :

les tensioactifs, les colorants, les agents dispersants, les conservateurs, les agents de sapidité, les arômes, les édulcorants, les anti-oxydants et leurs mélanges.

**17.** Médicament **caractérisé en ce qu'**il comprend de la suspension selon l'une quelconque des revendications 1 à 16.

**18.** Médicament **caractérisé en ce qu'**il comprend un nécessaire de préparation de la suspension selon l'une quelconque des revendications 1 à 16, lequel nécessaire comporte :

- des microcapsules sous forme sensiblement sèche contenant contenant l'amoxicilline pour permettre la saturation de la phase liquide en amoxicilline, une fois réalisée la mise en présence des deux phases solide et liquide;
- et/ou un mélange de microcapsules sous forme sensiblement sèche comprenant la dose d'amoxicilline juste nécessaire pour la libération modifiée ainsi qu'une dose nécessaire et suffisante d'amoxicilline non enrobé à libération immédiate, pour permettre la saturation de la phase liquide en d'amoxicilline, une fois réalisée la mise en présence de la dose de saturation d'amoxicilline et de la phase liquide;
- et la phase liquide et/ou au moins une partie des ingrédients utiles à sa préparation et/ou le protocole de préparation de la suspension.

**Claims**

**1.** Suspension of microcapsules in an aqueous liquid phase, said suspension being intended for oral administration and allowing the modified release of amoxicillin, **characterized in that**:

■ it comprises a plurality of microcapsules each consisting of a core containing amoxicillin and of a film coating that:

• is applied to the core,
• controls the modified release of the amoxicillin,
• and or has a composition corresponding to one of the following three families A or C:

⇒ <u>Family A</u>

♦ 1A - at least one film-forming polymer (P1) insoluble in the tract fluids, present in an amount of 50 to 90% and preferably of 50 to 80% by dry weight, based on the total weight of the coating composition, and consisting of at least one water-insoluble cellulose derivative;
♦ 2A - at least one nitrogen-containing polymer (P2) present in an amount of 2 to 25% and preferably of 5 to 15% by dry weight, based on the total weight of the coating composition, and consisting of at least one polyacrylamide and/or poly-N-vinylamide and/or poly-N-vinyllactam;
♦ 3A - at least one plasticizer present in an amount of 2 to 20% and preferably of 4 to 15% by dry weight, based on the total weight of the coating composition, and consisting of at least one of the following compounds: glycerol esters, phthalates, citrates, sebacates, cetyl alcohol esters and castor oil;
♦ 4A - at least one surfactant and/or lubricant present in an amount of 2 to 20% and preferably of 4 to 15% by dry weight, based on the total weight of the coating composition, and selected from anionic surfactants and/or non-ionic surfactants and/or lubricants, it being possible for said agent to comprise only one or a mixture of the above-mentioned products;

⇒ <u>Family C</u>

♦ 1C - at least one film-forming polymer insoluble in the gastrointestinal tract fluids;
♦ 2C - at least one water-soluble polymer;

♦ 3C - at least one plasticizer;
♦ 4C - optionally at least one surfactant/lubricant preferably selected from the following group of products:

~ anionic surfactants;
- and/or non-ionic surfactants,

■ and the liquid phase is saturated or becomes saturated with amoxicillin on contact with the microcapsules.

2. Suspension according to claim 1, **characterized in that** the families A and C of coating compositions are as follows:

⇒ Family A

♦ 1A - ethyl cellulose and/or cellulose acetate;
♦ 2A - polyacrylamide and/or polyvinylpyrrolidone;
♦ 3A - castor oil;
♦ 4A - an alkali metal or alkaline earth metal salt of fatty acids, stearic and/or oleic acid being preferred, a polyethoxylated sorbitan ester, a polyethoxylated castor oil derivative, a stearate, preferably calcium, magnesium, aluminium or zinc stearate, a stearylfumarate, preferably sodium stearylfumarate, or glycerol behenate, taken individually or in a mixture with one another;

⇒ Family C

♦ 1C

~ water-insoluble cellulose derivatives, ethyl cellulose and/or cellulose acetate being particularly preferred;
~ acrylic derivatives;
~ polyvinyl acetates;
~ and mixtures thereof;

♦ 2C

~ water-soluble cellulose derivatives;
~ polyacrylamides;
~ poly-N-vinylamides;
~ poly-N-vinyllactams;
~ polyvinyl alcohols (PVA);
~ polyoxyethylenes (POE);
~ polyvinylpyrrolidones (PVP) (the latter being preferred);
~ and mixtures thereof;

♦ 3C

~ glycerol and its esters, preferably from the following subgroup: acetylated glycerides, glycerol monostearate, glyceryl triacetate and glycerol tributyrate;
~ phthalates, preferably from the following subgroup: dibutyl phthalate, diethyl phthalate, dimethyl phthalate and dioctyl phthalate;
~ citrates, preferably from the following subgroup: acetyltributyl citrate, acetyltriethyl citrate, tributyl citrate and triethyl citrate;
~ sebacates, preferably from the following subgroup: diethyl sebacate and dibutyl sebacate;
~ adipates;
~ azelates;
~ benzoates;
~ vegetable oils;
~ fumarates, preferably diethyl fumarate;
~ malates, preferably diethyl malate;
~ oxalates, preferably diethyl oxalate;

~ succinates, preferably dibutyl succinate;
~ butyrates;
~ cetyl alcohol esters;
~ salicylic acid;
- triacetin;
- malonates, preferably diethyl malonate;
- cutin;
- castor oil (this being particularly preferred);
- and mixtures thereof;

♦ 4C

- alkali metal or alkaline earth metal salts of fatty acids, stearic and/or oleic acid being preferred;

~ polyethoxylated oils, preferably polyethoxylated hydrogenated castor oil;
polyoxyethylene/polyoxypropylene copolymers;
polyethoxylated sorbitan esters;
polyethoxylated castor oil derivatives;
stearates, preferably calcium, magnesium, aluminium or zinc stearate;
stearylfumarates, preferably sodium stearylfumarate;
glycerol behenate;
and mixtures thereof.

3. Suspension according to claim 1 or 2, **characterized in that** the film coating consists of a single layer.

4. Suspension according to claim 1, **characterized in that** it contains:

- 30 to 95% by weight and preferably 60 to 85% by weight of liquid phase (advantageously water);
- and 5 to 70% by weight and preferably 15 to 40% by weight of microcapsules.

5. Suspension according to claim 1, **characterized in that** the amount of solvent liquid phase (preferably water) for the amoxicillin is such that the proportion of dissolved amoxicillin originating from the microcapsules is less than or equal to 15% and preferably less than or equal to 5% by weight, based on the total weight of amoxicillin contained in the microcapsules.

6. Suspension according to claim 1, **characterized in that** the liquid phase is at least partially and preferably totally saturated with amoxicillin following the incorporation of the microcapsules into this liquid phase.

7. Suspension according to claim 6, **characterized in that** it is the active principle(s) contained in the microcapsules that saturate the liquid phase with amoxicillin.

8. Suspension according to claim 1, **characterized in that** the liquid phase is at least partially and preferably totally saturated with amoxicillin by means of non-encapsulated amoxicillin prior to the incorporation of the microcapsules into this liquid phase.

9. Suspension according to any one of claims 1 to 8, **characterized in that** the microcapsules have a particle size less than or equal to 1000 microns, preferably of between 200 and 800 microns and particularly preferably of between 200 and 600 microns.

10. Suspension according to any one of claims 1 to 9, **characterized in that** the film coating represents from 1 to 50% and preferably from 5 to 40% of the total weight of the coated microcapsules.

11. Suspension according to any one of claims 1 to 10, **characterized in that** the ingredients of said suspension are provided in an amount such that the said suspension has an in vitro release profile obtained using a type II apparatus according to the European Pharmacopoeia 3rd edition, in a phosphate buffer medium of pH 6.8 and at a temperature of 37°C, such that:

► the proportion PI of amoxicillin released during the first 15 minutes of the dissolution test is such that:

$$PI \leq 15$$

preferably PI ≤ 5;

► the remaining amoxicillin is released over a period such that the release time of 50% by weight of AP ($t_{1/2}$) is defined as follows (in hours):

$$0.5 \leq t_{1/2} \leq 30$$

preferably $0.5 \leq t_{1/2} \leq 20$.

12. Suspension according to any one of claims 1 to 11, **characterized in that in that** <u>the ingredient of said suspension are provided in an amount such that:</u>

   - the initial in vitro release profile Pfi obtained just after suspension of the microcapsules in the solvent (preferably aqueous) phase, using a type II apparatus according to the European Pharmacopoeia 3rd edition, in a phosphate buffer medium of pH 6.8, at a temperature of 37°C,
   - and the in vitro release profile $Pf_{10}$ obtained 10 days after suspension of the microcapsules in the solvent (preferably aqueous) phase, using a type II apparatus according to the European Pharmacopoeia 3rd edition, in a phosphate buffer medium of pH 6.8, at a temperature of 37°C,

   are similar.

13. Suspension according to any one of claims 1 to 12, **characterized in that** its pH is arbitrarily acidic or neutral.

14. Suspension according to any one of claims 1 to 13, **characterized in that** it comprises at least one rheology modifier.

15. Suspension according to any one of claims 1 to 14, **characterized in that** it comprises at least one agent for modifying the solubility of the amoxicillin in the solvent (preferably aqueous) liquid phase.

16. Suspension according to any one of claims 1 to 15, **characterized in that** it contains at least one additive selected from the group comprising surfactants, colourants, dispersants, preservatives, taste improvers, flavourings, sweeteners, antioxidants and mixtures thereof.

17. Drug, **characterized in that** it comprises a suspension according to any one of claims 1 to 16.

18. Drug, **characterized in that** it comprises a kit for preparing the suspension according to any one of claims 1 to 16, said kit containing:

   - microcapsules in substantially dry form containing containing amoxicillin for saturating the liquid phase with amoxicillin once the two solid and liquid phases have been brought into contact;
   - and/or a mixture of microcapsules in substantially dry form containing amoxicillin in the dose that is just necessary for modified release, together with immediate-release uncoated amoxicillin in a necessary and sufficient dose to saturate the liquid phase with amoxicillin once the saturation dose of amoxicillin and the liquid phase have been brought into contact;
   - and the liquid phase and/or at least part of the ingredients useful for its preparation, and/or the protocol for preparation of the suspension.

## Patentansprüche

1. Suspension von Mikrokapseln in einer wässrigen flüssigen Phase, wobei die Suspension zur oralen Verabreichung bestimmt ist und die modifizierte Freisetzung von Amoxicillin ermöglicht, **dadurch gekennzeichnet**:

   ■ dass sie eine Vielzahl von Mikrokapseln umfasst, die jeweils aus einem Kern, der Amoxicillin enthält, und einem Beschichtungsfilm besteht, der:

• auf den Kern aufgetragen wird,

• die modifizierte Freisetzung von Amoxicillin steuert,

• und eine Zusammensetzung hat, die einer der folgen-den Familien A oder C entspricht:

⇒ Familie A

♦ 1A - wenigstens ein filmbildendes Polymer (P1), das in den Flüssigkeiten des Magen-Darm-Trakts unlöslich ist und in einem Anteil von 50 bis 90 Gew.-%, vorzugsweise 50 bis 80 Gew.-%, Trockengewicht in Bezug auf die Gesamtmasse der Beschichtungszusammensetzung vorhanden ist und aus wenigstens einem wasserunlöslichen Cellulosederivat besteht;

♦ 2A - wenigstens ein stickstoffhaltiges Polymer (P2), das in einem Anteil von 2 bis 25 Gew.-%, vorzugsweise 5 bis 15 Gew.-%, Trockengewicht in Bezug auf die Gesamtmasse der Beschichtungszusammensetzung vorhanden ist und aus wenigstens einem Polyacrylamid und/oder Poly-N-vinylamid und/oder Poly-N-vinyllactam besteht;

♦ 3A - wenigstens ein Weichmacher, der in einem Anteil von 2 bis 20 Gew.-%, vorzugsweise 4 bis 15 Gew.-%, Trockengewicht in Bezug auf die Gesamtmasse der Beschichtungszusammensetzung vorhanden ist und aus wenigstens einer der folgenden Verbindungen besteht: Glycerinestern, Phthalaten, Citraten, Sebacaten, Estern von Cetylalkohol, Ricinusöl;

♦ 4A - wenigstens ein Tensid und/oder Gleitmittel, das in einem Anteil von 2 bis 20 Gew.-%, vorzugsweise 4 bis 15 Gew.-%, Trockengewicht in Bezug auf die Gesamtmasse der Beschichtungszusammensetzung vorhanden ist und aus anionischen Tensiden und/oder nichtionischen Tensiden und/oder Gleitmitteln ausgewählt ist, wobei das Tensid bzw. Gleitmittel ein einziges oder ein Gemisch der obigen Produkte umfassen kann;

⇒ Familie C

♦ 1C - wenigstens ein filmbildendes Polymer, das in den Flüssigkeiten des Magen-Darm-Trakts unlöslich ist;

♦ 2C - wenigstens ein wasserlösliches Polymer;

♦ 3C - wenigstens ein Weichmacher;

♦ 4C - und gegebenenfalls wenigstens ein Tensid/Gleitmittel, das vorzugsweise aus der Gruppe der folgenden Produkte ausgewählt ist:

~ anionischen Tensiden,

~ und/oder nichtionischen Tensiden;

■ und dass die flüssige Phase mit Amoxicillin gesättigt ist oder beim Kontakt mit den Mikrokapseln mit Amoxicillin gesättigt wird.

2. Suspension gemäß Anspruch 1, **dadurch gekennzeichnet, dass** es sich bei den Familien A und C der Beschich-tungszusammensetzung um Folgendes handelt:

⇒ Familie A

♦ 1A - Ethylcellulose und/oder Celluloseacetat;

♦ 2A - Polyacrylamid und/oder Polyvinylpyrrolidon;

♦ 3A - Ricinusöl;

♦ 4A - Alkalimetall- oder Erdalkalimetallsalz von Fettsäuren, wobei Stearinsäure und/oder Ölsäure bevorzugt sind, Polyoxyethylensorbitanester als Derivate von Polyoxyethylen-Ricinusöl, Stearat, vorzugsweise Calcium-, Magnesium-, Aluminium- oder Zinkstearat, Stearylfumarat, vorzugsweise Natriumstearylfumarat, Glycerinbehenat, jeweils allein oder im Gemisch;

⇒ Familie C

♦ 1C

~ nichtwasserlösliche Cellulosederivate, wobei Ethylcellulose und/oder Celluloseacetat besonders bevorzugt sind,

~ Acrylderivate,
~ Polyvinylacetate,
~ und ihre Gemische;

♦ 2C

~ wasserlösliche Cellulosederivate,
~ Polyacrylamide,
~ Poly-N-Vinylamide,
~ Poly-N-vinyllactame,
~ Polyvinylalkohole (PVA),
~ Polyoxyethylene (POE),
~ Polyvinylpyrrolidone (PVP) (wobei letztere bevorzugt sind),
~ und ihre Gemische;

♦ 3C

~ Glycerin und seine Ester, vorzugsweise aus der folgenden Untergruppe: acetylierte Glyceride, Glycerinmonostearat, Glyceryltriacetat, Glycerintributyrat,
~ Phthalate, vorzugsweise aus der folgenden Untergruppe: Dibutylphthalat, Diethylphthalat, Dimethylphthalat, Dioctylphthalat,
~ Citrate, vorzugsweise aus der folgenden Untergruppe: Acetyltributylcitrat, Acetyltriethylcitrat, Tributylcitrat, Triethylcitrat,
~ Sebacate, vorzugsweise aus der folgenden Untergruppe: Diethylsebacat, Dibutylsebacat,
~ Adipate,
~ Azelate,
~ Benzoate,
~ Pflanzenöle,
~ Fumarate, vorzugsweise Diethylfumarat,
~ Malate, vorzugsweise Diethylmalat,
~ Oxalate, vorzugsweise Diethyloxalat,
~ Succinate, vorzugsweise Dibutylsuccinat,
~ Butyrate,
~ Ester von Cetylalkohol,
~ Salicylsäure,
~ Triacetin,
~ Malonate, vorzugsweise Diethylmalonat,
~ Cutin,
~ Ricinusöl (wobei dieses besonders bevorzugt ist),
~ und ihre Gemische;

♦ 4C

~ Alkalimetall- oder Erdalkalimetallsalz von Fettsäuren, wobei Stearinsäure und/oder Ölsäure bevorzugt sind;
~ Polyoxyethylenierte Öle, vorzugsweise polyoxyethyleniertes hydriertes Ricinusöl;
Polyoxyethylen-Polyoxypropylen-Copolymere,
Polyoxyethylenierte Sorbitanester,
Polyoxyethylenierte Ricinusölderivate,
Stearate, vorzugsweise Calcium-, Magnesium-, Aluminium-oder Zinkstearat,
Stearylfumarate, vorzugsweise Natriumstearylfumarate, Glycerinbehenat,
und ihre Gemische.

3. Suspension gemäß Anspruch 1 oder 2, **dadurch gekennzeichnet, dass** der Beschichtungsfilm aus einer einzigen Schicht gebildet wird.

4. Suspension gemäß Anspruch 1, **dadurch gekennzeichnet, dass** sie Folgendes umfasst:

- 30 bis 95 Gew.-%, vorzugsweise 60 bis 85 Gew.-%, flüssige Phase (vorteilhafterweise Wasser),
- 5 bis 70 Gew.-%, vorzugsweise 15 bis 40 Gew.-%, Mikrokapseln.

5. Suspension gemäß Anspruch 1, **dadurch gekennzeichnet, dass** die Menge der flüssigen Phase (vorzugsweise Wasser), in der das Amoxicillin gelöst ist, so groß ist, dass der Anteil an gelöstem Amoxicillin und an Amoxicillin, das aus den Mikrokapseln stammt, kleiner oder gleich 15 Gew.-%, vorzugsweise 5 Gew.-%, ist, bezogen auf die Gesamtmasse an Amoxicillin, das in den Mikrokapseln enthalten ist.

6. Suspension gemäß Anspruch 1, **dadurch gekennzeichnet, dass** die flüssige Phase nach der Einarbeitung der Mikrokapseln in die flüssige Phase wenigstens teilweise, vorzugsweise vollständig, an Amoxicillin gesättigt ist.

7. Suspension gemäß Anspruch 6, **dadurch gekennzeichnet, dass** die Amoxicillinsättigung mittels des oder der Wirkstoffe erfolgt, der bzw. die in den Mikrokapseln enthalten sind.

8. Suspension gemäß Anspruch 1, **dadurch gekennzeichnet, dass** die flüssige Phase vor der Einarbeitung der Mikrokapseln in die flüssige Phase mit Hilfe von nicht eingekapseltem Amoxicillin wenigstens teilweise, vorzugsweise vollständig, an Amoxicillin gesättigt ist.

9. Suspension gemäß einem der Ansprüche 1 bis 8, **dadurch gekennzeichnet, dass** die Mikrokapseln eine Teilchengröße von kleiner oder gleich 1000 $\mu$m haben, die vorzugsweise zwischen 200 und 800 $\mu$m und besonders bevorzugt zwischen 200 und 600 $\mu$m liegt.

10. Suspension gemäß einem der Ansprüche 1 bis 9, **dadurch gekennzeichnet, dass** der Beschichtungsfilm 1 bis 50 Gew.-%, vorzugsweise 5 bis 40 Gew.-%, der Gesamtmasse der beschichteten Mikrokapseln ausmacht.

11. Suspension gemäß einem der Ansprüche 1 bis 10, **dadurch gekennzeichnet, dass** die Bestandteile der Suspension in einer solchen Menge vorgesehen sind, dass die Suspension in vitro ein Freisetzungsprofil, das mit Hilfe einer Apparatur des Typs II gemäß dem Europäischen Arzneibuch, 3. Auflage, in einem phosphatgepufferten Medium, pH 6,8, und bei einer Temperatur von 37 °C bestimmt wurde, der folgenden Art hat:

▶ der Anteil PI an Amoxicillin, das während der ersten 15 Minuten des Auflösungstests freigesetzt wird, beträgt:

$$PI \leq 15$$

vorzugsweise

$$PI \leq 5,$$

▶ die Freisetzung des übrigen Amoxicillins erfolgt über einen solchen Zeitraum, dass die Freisetzungszeit für 50 Gew.-% PA ($t_{1/2}$) wie folgt definiert (in Stunden):

$$0,5 \leq t_{1/2} \leq 30$$

vorzugsweise

$$0,5 \leq t_{1/2} \leq 20.$$

12. Suspension gemäß einem der Ansprüche 1 bis 11, **dadurch gekennzeichnet, dass** die Bestandteile der Suspension in einer solchen Menge vorgesehen sind, dass:

- das in-vitro-Anfangsfreisetzungsprofil Pfi, das unmittelbar nach der Suspendierung der Mikrokapseln in der (vorzugsweise wässrigen) Lösungsmittelphase mit Hilfe einer Apparatur des Typs II gemäß dem Europäischen Arzneibuch, 3. Auflage, in einem phosphatgepufferten Medium, pH 6,8, und bei einer Temperatur von 37 °C bestimmt wurde,

- und das in-vitro-Freisetzungsprohl $Pf_{10}$, das 10 Tage nach der Suspendierung der Mikrokapseln in der (vorzugsweise wässrigen) Lösungsmittelphase mit Hilfe einer Apparatur des Typs II gemäß dem Europäischen Arzneibuch, 3. Auflage, in einem phosphatgepufferten Medium, pH 6,8, und bei einer Temperatur von 37 °C bestimmt wurde,

gleichartig sind.

13. Suspension gemäß einem der Ansprüche 1 bis 12, **dadurch gekennzeichnet, dass** ihr pH-Wert entweder sauer oder neutral ist.

14. Suspension gemäß einem der Ansprüche 1 bis 13, **dadurch gekennzeichnet, dass** sie wenigstens einen Rheologiemodifikator umfasst.

15. Suspension gemäß einem der Ansprüche 1 bis 14, **dadurch gekennzeichnet, dass** sie wenigstens einen Modifikator der Löslichkeit des Amoxicillins in der flüssigen (vorzugsweise wässrigen) Lösungsmittelphase umfasst.

16. Suspension gemäß einem der Ansprüche 1 bis 15, **dadurch gekennzeichnet, dass** sie wenigstens ein Additiv umfasst, das aus der Gruppe ausgewählt ist, die Folgende umfasst:

Tenside, Färbemittel, Dispergiermittel, Konservierungsstoffe, Geschmacksstoffe, Aromen, Süßungsmittel, Antioxidantien und Gemische davon.

17. Medikament, **dadurch gekennzeichnet, dass** es die Suspension gemäß einem der Ansprüche 1 bis 16 umfasst.

18. Medikament, **dadurch gekennzeichnet, dass** es einen Kit für die Herstellung der Suspension gemäß einem der Ansprüche 1 bis 16 umfasst, wobei der Kit Folgendes umfasst:

- Mikrokapseln in einer im Wesentlichen trockenen Form, die Amoxicillin enthalten und die Sättigung der flüssigen Phase an Amoxicillin ermöglichen, sobald die feste und die flüssige Phase miteinander in Kontakt gebracht wurden;
- und/oder ein Gemisch von Mikrokapseln in einer im Wesentlichen trockenen Form, die die für die modifizierte Freisetzung gerade notwendige Dosis an Amoxicillin sowie eine notwendige und hinreichende Dosis an nicht beschichtetem Amoxicillin für die sofortige Freisetzung umfassen und die Sättigung der flüssigen Phase an Amoxicillin ermöglichen, sobald die Dosis für die Amoxicillinsättigung und die flüssige Phase miteinander in Kontakt gebracht wurden;
- und die flüssige Phase und/oder wenigstens einen Teil der Bestandteile, die zu ihrer Herstellung dienen, und/oder die Anweisung zur Herstellung der Suspension.

**Figure 1**

**Figure 2**

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- EP 0709087 B
- WO 9427557 A
- WO 9520946 A
- WO 9528148 A
- WO 9604908 A
- WO 0061115 A
- WO 0061116 A
- WO 0147499 A
- WO 0230392 A
- WO 9835672 A
- WO 0050036 A
- WO 9706798 A
- WO 0003695 A
- WO 9836732 A
- WO 9709042 A
- WO 8707833 A
- US 4902513 B
- EP 0601508 A
- WO A9601628 A
- EP 1062955 A
- WO 9119486 A
- EP 0273890 A
- EP 0953359 B

**Littérature non-brevet citée dans la description**

- pharmacopée européenne
- **Arthur H KIBBE.** Handbook of pharmaceutical excipients. Am. Pharmaceutical Association, 2000
- **C. DUVERNEY ; J.P. BENOIT.** *L'actualité chimique,* Décembre 1986
- pharmacopée Européenne